Europäisches Patentamt

European Patent Office

Office européen des brevets

(11) Veröffentlichungsnummer: **0 543 152 A2**

# EUROPÄISCHE PATENTANMELDUNG

(21) Anmeldenummer: **92117770.5**

(22) Anmeldetag: **18.10.92**

(51) Int. Cl.5: **A61N 5/00, A61N 1/40**

(30) Priorität: **17.10.91 SU 5004438**

(43) Veröffentlichungstag der Anmeldung:
**26.05.93 Patentblatt 93/21**

(84) Benannte Vertragsstaaten:
**AT BE CH DE DK ES FR GB GR IE IT LI LU MC NL PT SE**

(71) Anmelder: **Ortlib, Sergej**
**Weserring 9**
**W- 3501 Guxhagen(DE)**
Anmelder: **Malzew, Alexander P.**
**Ul Tschajkowskaja 70/60**
**454015 Celjabinsk(RU)**

(72) Erfinder: **Darowskich, Stanislav Nikiforovitsch**
**Gorodok II Haus 28, Wohnung 12 (II)**
**SU- 454015 Celjabinsk- 15(SU)**
Erfinder: **Safin, Dajan Katipovitsch**

**Gorodok II Haus 34, Wohnung 85 (II)**
**SU- 454015 Celjabinsk- 15(SU)**
Erfinder: **Ovsjannikov, Nikolaij Witaljevitsch**
**Gorodok II Haus 5, Wohnung 15 (II)**
**SU- 454015 Celjabinsk- 15(SU)**
Erfinder: **Pustaserov, Oleg Wladimirovitsch**
**Kuischguimskaja Haus 12a, Wohnung 6 (II)**
**SU- 454008 Celjabinsk- 8(SU)**
Erfinder: **Tschernjakov, Gennadij**
**Michailovitsch**
**ul. Marschala Novikova Haus 3, Wohnung**
**83(II)**
**SU- 196349 St. Petersburg- 349(su)**

(74) Vertreter: **Freiherr von Schorlemer, Reinfried,**
**Dipl.- Phys.**
**Patentanwalt Brüder- Grimm- Platz 4**
**W- 3500 Kassel (DE)**

(54) **Vorrichtung zur Stimulation des funktionellen Zustands eines biologischen Objekts.**

(57) Es wird eine Vorrichtung für die Stimulation des Funktionszustands eines biologischen Objekts beschrieben, die sich beispielsweise zur Behandlung des Bronchialasthmas beim Menschen eignet. Die Vorrichtung besteht aus einem UHF- Generator (1), dem eine Modulationseinrichtung (2) zugeordnet ist. An den Ausgang des UHF- Generators (1) ist eine Antenneneinrichtung (3) angeschlossen, die zur Abstrahlung eines höchstfrequenten, elektromagnetischen Signals dient. Die Modulationseinrichtung (2) zeichnet sich dadurch aus, daß sie einen nach einem pseudozufälligen Gesetz arbeitenden Frequenz- und/oder Amplitudenmodulator (6,7) enthält.

Fig. 1

EP 0 543 152 A2

Die Erfindung betrifft eine Vorrichtung der im Oberbegriff des Anspruchs 1 angegebenen Gattung.

Vorrichtungen dieser Art dienen zur elektromagnetischen Einwirkung auf biologische Objekte, insbesondere z.B. auf die Zellen von Pflanzen, Säugetieren oder auch Menschen. Dabei wird versucht, mit Hilfe von Höchstfrequenzfeldern derart auf die biologischen Objekte einzuwirken, daß ausgewählte Funktionen wie z.B. das Wachstum oder das Aufkeimen von Pflanzen stimuliert werden. Auch zur Stimulation der Lokalhyperthermie (AS. Belakowskij in "Biophysik in der Veterinärlehre" S. 179 − 194, Agrowirtschaftlicher Verlag Moskau 1989) oder der Resistenz von Tieren sind derartige Vorrichtungen bekannt.

Bei einer bekannten Vorrichtung der eingangs bezeichneten Art (US 4 621 642), die zur Einwirkung auf ausgewählte Akupunkturpunkte dient, die sich in begrenzten Abschnitten auf der Oberfläche einiger biologischer Objekte befinden, erfolgt die Stimulation in bestimmten, bevorzugten Frequenzbereichen und z.B. bei 400 − 500 MHz mit größter Intensität.

Bei einer anderen bekannten Vorrichtung der genannten Art (US 4 877 027) geht es dagegen um die Stimulation des menschlichen Gehirns mit einem gepulsten Höchstfrequenzsignal in der Absicht, von den Hörorganen unabhängige Schalleindrücke zu erzeugen. Dazu werden Impulsserien des Trägersignals mit konstanter Radiofrequenz frequenzmoduliert, wobei die Einwirkungszeiten der Impulsserien zwischen einer Nanosekunde und zehn Mikrosekunden variiert werden.

Ein gemeinsamer Nachteil der beiden bekannten Vorrichtungen besteht in der hohen Adaptionsfähigkeit der biologischen Objekte an die einwirkenden Strahlungen, wodurch deren Wirkung gering bzw. von kurzer Dauer ist.

Der Erfindung liegt die Aufgabe zugrunde, die eingangs bezeichnete Vorrichtung so auszubilden, daß die Wirksamkeit der Stimulation des funktionellen Zustands eines biologischen Objekts wesentlich verbessert wird.

Zur lösung dieser Aufgabe dienen die kennzeichnenden Merkmale des Anspruchs 1.

Die Erfindung bringt den wesentlichen und überraschenden Vorteil mit sich, daß durch die sich ständig ändernde Art der Frequenz− und/oder Amplitudenmodulation eine schnelle Adaption des stimulierten biologischen Objekts an die elektromagnetische Strahlung verhindert und dadurch eine wesentliche Vergrößerung der Wirksamkeit der Vorrichtung erzielt wird. Dabei ist gleichzeitig eine Verringerung der Intensität der Strahlung bei der Anwendung möglich. Weiterhin kann mit den UHF−Signalen auch auf das vegetative Nervensystem des Menschen eingewirkt werden, d.h. es entfällt das Erfordernis, auf der Oberfläche des biologischen Objekts bestimmte Punkte aufzusuchen. Als besonders erfolgreich hat sich die erfindungsgemäße Vorrichtung überraschend bei der Behandlung des Bronchialasthmas erwiesen, wenn die elektromagnetische Strahlung auf die vordere Fläche des unteren Halsteils und auf den Brustkorb in Höhe des 6. Hals− bzw. 3. Brustwirbels der Patienten gerichtet wird. Die Trägerfrequenz sollte dabei nicht unter 3,5 GHz und die Frequenzänderung bei der pseudozufälligen Frequenzmodulation bei nicht mehr als 500 MHz liegen. Die Einwirkung wird vorzugsweise im nicht warmen Bereich mit einer Strählungsdichte von nicht mehr als 100 mWt/cm$^2$ und einer Einwirkungsdauer von nicht mehr als 600 Sekunden realisiert. Eine zusätzliche Amplitudenmodulation wird vorzugsweise nach einem Sinusgesetz harmonisch oder impulsweise im Frequenzbereich von 5 − 500 Hz bei einem Modulationskoeffizienten von 0 − 100 % durchgeführt.

Weitere vorteilhafte Merkmale der Erfindung ergeben sich aus den Unteransprüchen.

Die Erfindung wird nachfolgend in Verbindung mit der beiliegenden Zeichnung an einem Ausführungsbeispiel näher erläutert. Es zeigen:

| | |
|---|---|
| Fig. 1 | ein Blockschaltbild der erfindungsgemäßen Vorrichtung; |
| Fig. 2 | ein Schaubild mit dem Verlauf von mehreren, zur Frequenzmodulation verwendeten Spannungen; |
| Fig. 3 | eine Steuereinrichtung zur Erzeugung der Spannungen nach Fig. 2; |
| Fig. 4 | ein Impulsdiagramm der Steuervorrichtung nach Fig. 3; |
| Fig. 5 | ein Schaubild mit dem Verlauf der die Frequenzmodulation bewirkenden Spannungen; |
| Fig. 6 | schematisch einen UHF−Generator der erfindungsgemäßen Vorrichtung; und |
| Fig. 7 | ein Schaubild mit dem Verlauf der amplitudenmodulierten Spannung der erfindungsgemäßen Vorrichtung. |

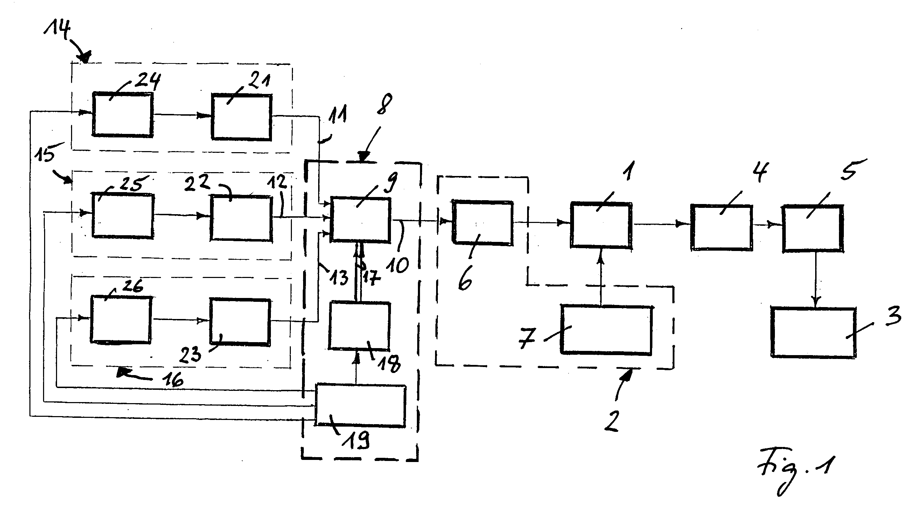

Nach Fig. 1 enthält eine erfindungsgemäße Vorrichtung für die Stimulation des funktionellen Zustands eines biologischen Objekts einen Ultrahochfrequenzgenerator 1, der nachfolgend kurz UHF−Generator bezeichnet wird. Dem UHF−Generator 1 ist erfindungsgemäß eine Modulationseinrichtung 2 zugeordnet. Außerdem ist dem UHF−Generator 1 eine Antenneneinrichtung 3 nachgeschaltet, die z.B. eine Spiralantenne enthält wobei zwischen den UHF−Generator 1 und die Antenneneinrichtung 3 zweckmäßig noch ein Frequenzvervielfacher 4 und ein beispielsweise als Kapazitätsverstärker ausgebildeter Verstärker 5 geschaltet sind.

Die Modulationseinrichtung 2 enthält einen Frequenzmodulator 6 und/oder auch einen Amplitudenmodulator 7, vorzugsweise insbesondere den Frequenzmodulator 6. Dabei steht der Frequenzmodulator 6 unter der Steuerung einer an seinen Eingang angeschlossenen Steuervorrichtung 8, die zufällig bzw. pseudozufällig arbeitet und für eine sich ständig ändernde Frequenzmodulation des UHF-Generators 1 sorgt. Die Steuervorrichtung 8 enthält einen Schalter 9, der einen an den Frequenzmodulator 6 angeschlossenen Ausgang 10 und wenigstens zwei, im Ausführungsbeispiel drei Eingänge 11, 12 und 13 aufweist, die wahlweise mit dem Ausgang 10 verbunden werden können. An jedem der Eingänge liegt der Ausgang eines Spannungsgenerators 14, 15 bzw. 16 mit steuerbarer Frequenz, und mehrere, hier z.B. zwei, Steuereingänge 17 des Schalters 9 sind mit zugehörigen Ausgängen einer Schalteinrichtung 18 verbunden, welche die Zeitpunkte, zu denen der Ausgang 10 mit einem der Eingänge 11 bis 13 verbunden wird, und die Zeitintervalle steuert, während denen diese Verbindungen aufrechterhalten bleiben. Schließlich enthält die Steuervorrichtung 8 einen Steuerblock 19, der für die Steuerung der gesamten Vorrichtung zuständig ist und daher, wie Fig. 1 schematisch zeigt, z.B. mit den Spannungsgeneratoren 14 bis 16 und mit der Schalteinrichtung 18 verbunden wird.

Die Spannungsgeneratoren 14 bis 16 enthalten vorzugsweise je einen Funktionsgenerator 21, 22 bzw. 23 mit steuerbarer Frequenz, dessen Ausgang an den entsprechenden Eingang 11, 12 bzw. 13 des Schalters 9 angeschlossen ist, und einen Spannungserzeuger 24, 25 bzw. 26, dessen Ausgang mit dem Eingang eines zugeordneten Funktionsgenerators 21, 22 bzw. 23 verbunden ist und der unter der Steuerung des Steuerblocks 19 steht.

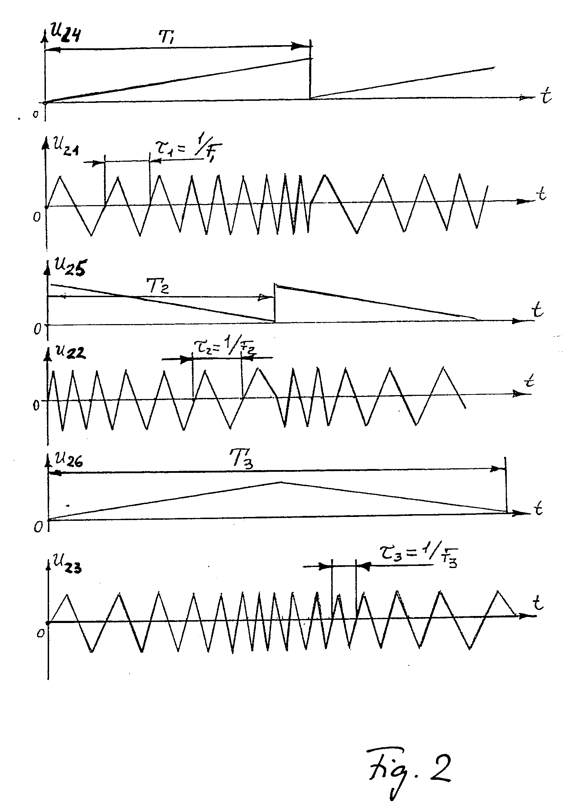

Fig. 2 zeigt die an den Ausgängen der Blöcke 21 bis 23 bzw. 24 bis 26 erscheinenden Spannungen $U_{21}$ bis $U_{26}$ in Abhängigkeit von der Zeit t. Danach gibt der Spannungserzeuger 24 eine mit der Zeit allmählich ansteigende Sägezahnspannung $U_{24}$, der Spannungserzeuger 25 eine mit der Zeit allmählich abfallende Sägezahnspannung $U_{25}$ und der Spannungserzeuger 26 eine zunächst allmählich ansteigende und dann allmählich absinkende Dreieckspannung $U_{26}$ ab, wobei die Perioden $T_1$ bis $T_3$ im Steuerblock 19 z.B. mit Hilfe von RC-Gliedern eingestellt werden.

Die von den Funktionsgeneratoren 21 bis 23 abgegebenen Spannungen $U_{21}$ bis $U_{23}$ ändern ihre Frequenzen $F_1$ bis $F_3$ proportional zu den Amplituden der Eingangsspannungen $U_{24}$ bis $U_{26}$, wie in Fig. 2 schematisch gezeigt ist, so daß an den Eingängen 11, 12 und 13 des Schalters 9 Spannungen liegen, die einen ganz unterschiedlichen Frequenzverlauf haben. Dabei werden die Frequenzen bzw. Perioden $\tau_1$, $\tau_2$ und $\tau_3$ vom Steuerblock 19 aus gesteuert und bei Bedarf auch ständig verändert, wobei die Frequenzen $F_1$ bis $F_3$ z.B. zwischen 20 Hz und 20 kHz liegen und die Frequenzänderungen $dF_1/dt$, $dF_2/dt$ und $dF_3/dt$ z.B. ± (5 − 200) • 1000 Hz/sek betragen.

Spannungserzeuger 24 bis 26 (vgl. z.B. B.I. Gorschkov in ''Radioelektronische Vorrichtungen'', Nachschlage-Buch, Moskau, Radio und Verbindung 1984, Bild 11.5 auf S. 260 für 24,25 und Bild 11.13 auf S. 265 für 26) sowie Funktionsgeneratoren 21 bis 23 (vgl. z.B. P. Chroviz/U. Chill in "Kunst der Schematechnik" in der englischen Übersetzung von M. Mir, 1984, Band 1, S. 227, Bild 3.67 und Band 2, S. 467) der beschriebenen Art sind allgemein bekannt und brauchen daher nicht näher erläutert werden.

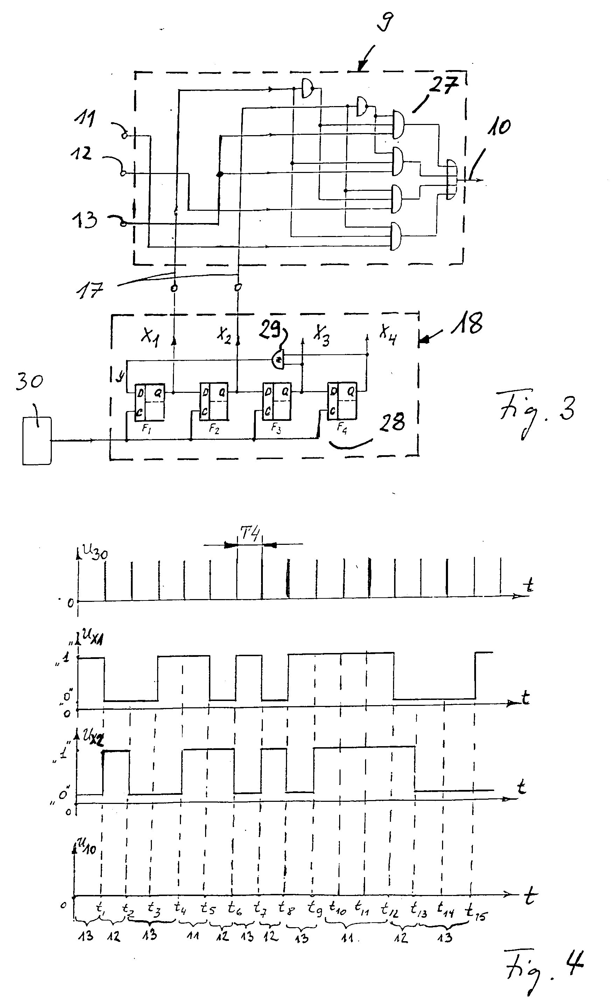

Die Steuervorrichtung 8 ist vorzugsweise entsprechend Fig. 3 ausgebildet. Sie enthält als Schalter 9 einen Multiplexer 27 und als Schalteinrichtung ein 4 Bit-Schieberegister 28 mit vier Ausgängen $X_1$, $X_2$, $X_3$ und $X_4$, wobei die beiden ersten Ausgänge $X_1$ und $X_2$ mit den beiden Steuereingängen 17 des Schalters 9 verbunden sind, während die beiden letzten Ausgänge $X_3$ und $X_4$ über ein exklusives ODER-Glied 29 mit dem Informationseingang Y des Schieberegisters 28 verbunden sind. Der Takteingang des Schieberegisters ist vorzugsweise mit einem üblichen Multivibrator 30 verbunden, der Teil des Steuerblocks 19 ist. Die Informationseingänge des Multiplexers 27 sind gleichzeitig die Eingänge 11 bis 13 des Schalters 9, wobei, wie in Fig. 3 gezeigt ist, zwei Informationseingänge miteinander verbunden sind und z.B. gemeinsam den Eingang 13 bilden.

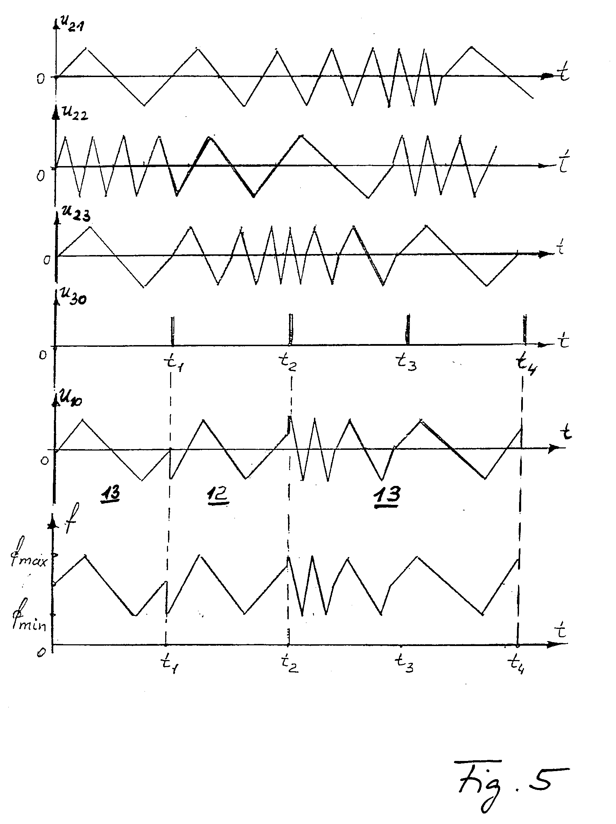

Die Spannungen $U_{30}$, $U_{X1}$, $U_{x2}$ und $U_{10}$, die am Ausgang des Multivibrators 30, an den Ausgängen $X_1$ und $X_2$ des Schieberegisters 28 und am Ausgang 10 des Schalters 9 entstehen, sind in Fig. 4 dargestellt. Danach gibt der Multivibrator 30 Taktsignale mit der Periode $T_4$ ab, die den Takteingängen C der das Schieberegister 28 bildenden D-Flipflops zugeführt werden. Dies hat zur Folge, daß die am Informationseingang D jedes Flipflops vorhandene Information (binäres "0"- oder "1"-Signal) beim Erscheinen des Taktsignals zum zugehörigen Ausgang $X_1$ bis $X_4$ geschoben wird, d.h. eine am Eingang D des ersten Flipflop vorhandene Information wird bei jedem Takt um eine Stufe weitergeschoben und dem Eingang des nächsten Flipflop zugeführt, wobei der Signalfluß in Fig. 3 von links nach rechts verläuft.

Zur Erzeugung von pseudozufälligen Impulsfolgen an den Ausgängen $X_1$ und $X_2$ wird davon ausgegangen, daß vor dem Erscheinen des ersten Taktimpulses ein vorgewählter Binärcode im Schieberegister 28 hergestellt wurde, beispielsweise in der Weise, daß sich der Ausgang $X_1$ auf

logisch "1" befindet, während die Ausgänge $X_2$ bis $X_4$ auf logisch "0" liegen, wie in Fig. 4 für das Zeitintervall von 0 bis $t_1$ angedeutet ist. Der erste Taktimpuls vom Multivibrator 30 (Zeitpunkt $t_1$ in Fig. 4) bewirkt dann, daß der Ausgang $X_2$ auf "1" gesetzt wird und gleichzeitig $X_1 = X_3 = X_4 = 0$ ist. Der nächste Taktimpuls ($t_2$) hat $X_3 = 1$ und $X_1 = X_2 = X_4 = 0$ zur Folge. Der dritte Takt ($t_3$) verursacht $X_1 = X_4 = 1$ und $X_2 = X_3 = 0$, weil zu diesem Zeitpunkt wegen des ODER−Gliedes 29 Y $= 1$ ist. Entsprechend hat der vierte Taktimpuls $X_1 = X_2 = 1$ und $X_3 = X_4 = 0$ zur Folge, während der nächste Takt ($t_5$) zu $X_2 = X_3 = 1$ und $X_1 = X_4 = 0$ und der sechste Takt ($t_6$) zu $X_1 = X_3 = X_4 = 1$ und $X_2 = 0$ führt. Der siebente Taktimpuls ($t_7$ bewirkt dann $X_1 = X_3 = 0$ und $X_2 = X_4 = 1$, weil wegen der exklusiven ODER−Funktion bei $X_3 = X_4 = 1$ gleichzeitig $Y = 0$ ist. Ähnliche Wechsel der Impulse an den Ausgängen $X_1$ bis $X_4$ ergeben sich zu den Zeitpunkten $t_8$ bis $t_{15}$. Der insgesamt fünfzehnte Takt ($t_{15}$) stellt wieder denjenigen Zustand her, der für das Zeitintervall von 0 bis $t_1$ beschrieben wurde, und danach wiederholt sich die beschriebene Impulsfolge. Weil während der Zeit zwischen $t_1$ und $t_{15}$ an den Ausgängen $X_1$ und $X_2$ Impulse in völlig chaotischer Reihenfolge ohne jede Regelmäßigkeit erscheinen, kann dieses Verhalten "pseudozufällig" bzw. "zufällig" bezeichnet werden.

In der untersten Zeile der Fig. 4 ist schematisch angedeutet, von welchem Eingang des Schalters 9 zu den verschiedenen Zeitpunkten $t_1$ bis $t_{15}$ die an dessen Ausgang 10 erscheinende Spannung $U_{10}$ stammt. Dabei wird davon ausgegangen, daß jede Kombination von Ausgangssignalen an den Ausgängen $X_1$ und $X_2$ jeweils eine Adresse bedeutet, d.h. die Signalkombinationen 10, 01, 00 und 11 an $X_1$, $X_2$ ergeben vier Adressen, die im Multiplexer 27 zur Folge haben, daß die zugehörigen Eingänge 11 bis 13 in Abhängigkeit von einer zugeordneten Adresse mit dem Ausgang 10 verbunden werden. Bei der beispielsweisen Darstellung gilt, daß der Ausgang 10 bei $X_1 = X_2 = 1$ mit dem Eingang 11, bei $X_1 = 0$, $X_2 = 1$ mit dem Eingang 12 und bei $X_1 = X_2 = 0$ und $X_1 = 1$, $X_2 = 0$ mit dem Eingang 13 verbunden ist, wie unterhalb der Zeitachse der untersten Zeile der Fig. 4 jeweils angegeben ist. Außerdem versteht sich, daß das Zeitintervall, währenddessen die mit dem Ausgang 10 verbundenen Eingänge chaotisch wechseln, $N = 2^n - 1$ Taktimpulse enthält, wobei n die Zahl der Stufen (hier D−Flipflops) des Schieberegisters 28 ist, d.h. bei zehn Stufen würde die Periode der pseudozufällig erscheinenden Spannungskombinationen $U_{X1}$, $U_{X2}$ schon 1023 betragen. Abgesehen davon kann natürlich auch von beliebigen anderen Anfangszuständen im Schieberegister 28 mit Ausnahme von $X_1 = X_2 =$

$X_3 = X_4 = 0$ ausgegangen werden.

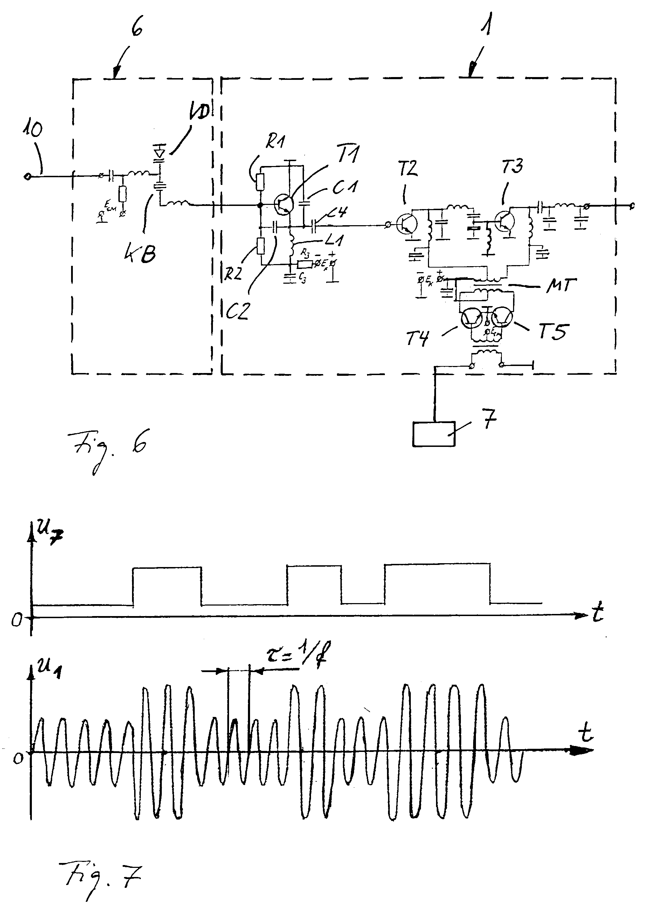

Die Spannung $U_{10}$, die entsprechend der beschriebenen Betätigung des Schalters 9 tatsächlich an dessen Ausgang 10 erscheint, ist in der fünften Zeile der Fig. 5 dargestellt. Dabei sind in den ersten drei Zeilen der Fig. 5 noch einmal die bereits in Fig. 2 dargestellten, an den Eingängen 11 bis 13 liegenden Spannungen $U_{21}$, $U_{22}$ und $U_{23}$ dargestellt, allerdings in vergrößertem Maßstab und nur für die Dauer von vier Taktimpulsen (Spannung $U_{30}$ in der vierten Zeile der Fig. 5). Daraus ergibt sich analog zu Fig. 4, daß z.B. von $t_0$ bis $t_1$ und von $t_2$ bis $t_4$, wenn der Eingang 13 mit dem Ausgang 10 verbunden ist, die Spannung $U_{23}$ am Ausgang 10 erscheint, während zwischen $t_1$ und $t_2$ die Spannung $U_{22}$ am Ausgang 10 liegt, weil jetzt der Eingang 12 mit dem Ausgang 10 verbunden ist. Die Spannung $U_{10}$ hat daher ständig bzw. "pseudozufällig" wechselnde Frequenzen und dementsprechend wechselnde momentane Amplituden. Außerdem ist die Länge der Einschaltung jedes Eingangs 11 bis 13 an den Ausgang 10 ebenfalls durch ein pseudozufälliges Gesetz bestimmt und gleich dem Einfachen oder einem Vielfachen der Periode $T_4$ der Taktimpulse (Fig. 4), die sich z.B. zwischen $T_4 = 0,01$ sek und $T_4 = 1$ sek ändern kann.

Der in Fig. 1 dargestellte UHF−Generator 1 ist ein Generator, der bei fehlenden bzw. unwirksam gemachten Modulatoren 6 und 7 an seinem Ausgang ein Radiosignal mit konstanter Frequenz und Amplitude abgeben würde.

Nach Fig. 6 ist zwischen den Schalter 9 und den UHF−Generator 1 ein Frequenzmodulator 6 geschaltet, der ein mit einer Kapazitätsdiode VD (Varicap) versehener, an sich bekannter Baustein ist (M.S. Schumilin, O.W. Golovin in "Radiosendervorrichtungen",Moskau, Hochschule, 1981, S. 227 − 229, Bilder 12.13 oder 12.15). Dabei hat die Kapazitätsdiode VD die Eigenschaft, ihre Kapazität in Abhängigkeit von der momentanen Amplitude des Eingangssignals, das dem Ausgangssignal des Schalters 9 am Ausgang 10 entspricht, zu ändern. Außerdem ist der Frequenzmodulator 6 so ausgebildet, däß die momentane Kapazität der Kapazitätsdiode VD die momentane Frequenz des UHF−Generators 1 festlegt.

Hierzu enthält der UHF−Generator 1 einen Transistor T1, dessen Basis über eine Steuerspule L2 und einen Schwingquarz KB des Frequenzmodulators 6 mit dessen Kapazitätsdiode VD verbunden ist. Widerstände R1, R2 und R3 versorgen den Transistor T1 in üblicher Weise mit Gleichstrom, während Kondensatoren C3, C4 und C6 und eine Drossel L1 als blockierende Elemente bzw. Filter wirken.

Der Transistor T1 bildet einen Schwingkreis, dessen Resonanzschwingungen eine vom Schwingquarz KB stabilisierte Frequenz haben und der außerdem eine Kondensatoreinheit mit den Kondensatoren C1, C2 und der Kapazität der Kapazitätsdiode VD in der Weise aufweist, daß die Modulationsfrequenz der Resonanzschwingungen des Generators von den Änderungen der Kapazität der Kapazitätsdiode VD infolge einer Änderung der Spannung $U_{10}$ abhängt. Da andererseits die Frequenz umgekehrt proportional der Wurzel aus dem Produkt von L und C ist, wird die Resonanzfequenz tatsächlich von der momentanen Amplitude der Spannung $U_{10}$ vorgegeben, so daß die Frequenz einen Verlauf entsprechend der untersten Zeile in Fig. 5 hat.

Im übrigen wird die Modulationsspannung $U_{10}$ über einen den Kondensator C6 und eine Spule L3 enthaltenden Schaltkreis an den PN−Übergang der Kapazitätsdiode VD gelegt, dem außerdem über einen Widerstand R4 und die Spule L3 eine konstante Spannung $E_{CM}$ zugeführt wird, die so eingestellt ist, daß stets $E_{CM} > U_{10}$ und daher der PN−Übergang stets gesperrt ist.

Die Amplitudenmodulation des vom Transistor T1 abgegebenen Signals erfolgt mit Hilfe eines an den Ausgang des Transistors T1 angeschlossenen, an sich bekannten Bausteins, der weitere Transistoren T2, T3, T4 und T5 enthält (M.S. Schumilin, O.W. Golovin in "Radiosendervorrichtungen", Moskau, Hochschule, 1981, S. 216, Bild 11.21). Dabei dienen die Transistoren T2 und T3 der Verstärkung des vom Transistor T1 generierten und mittels der Kapazitätsdiode VD frequenzmodulierten Signals. Zur Amplitudenmodulation dieses Signals dient ein Modulationstransformator MT, dem eine von den Transistoren T4 und T5 verstärkte, vom Amplitudenmodulator 7 abgegebene Modulationsspannung $U_7$ zugeführt wird. Dabei kann der Amplitudenmodulator 7 ein Multivibrator sein, der Impulse mit konstanter Frequenz und Dauer abgibt. Bevorzugt handelt es sich jedoch um eine Einrichtung mit steuerbarer Impulsbreite, z.B. einen an einen Oszillator angeschlossenen Univibrator, und möglichst auch um eine Einrichtung, die ein Signal abgibt, dessen Impulsfolgefrequenz und/oder Amplitude steuerbar ist, wobei diese Steuerung analog zu Fig. 1 mit Hilfe des Steuerblocks 19 vorgenommen werden könnten. Möglich wäre auch, die genannten Größen analog zur Frequenzmodulation mit Hilfe eines Zufallsgenerators bzw. einer pseudozufällig generierten Impulsfolge zu steuern, um dadurch eine schnelle Adaption des biologischen Objekts an die elektromagnetische Strahlung ganz oder teilweise mit Hilfe der Amplitudenmodulation zu verhindern.

Die Modulationsspannung $U_7$ ist in Fig. 7 schematisch als eine Spannung dargestellt, die Impulse mit unterschiedlicher Folgefrequenz und mit unterschiedlicher Breite abgibt. Dementsprechend ist das Ausgangssignal $U_1$ des UHF−Generators 1 auch amplitudenmoduliert, wobei in Fig. 7 zur Vereinfachung der Darstellung angenommen ist, daß das UHF−Signal in der in Fig. 7 betrachteten Zeitspanne keiner gleichzeitigen Frequenzmodulation unterliegt. Tatsächlich ist das Signal $U_1$ jedoch sowohl entsprechend Fig. 5 in der Frequenz als auch entsprechend Fig. 7 in der Amplitude moduliert.

Im Frequenzvervielfacher 4 wird die Frequenz des Ausgangssignals des UHF−Generators 1 mit einem Faktor n multipliziert, wobei z.B. n = 40 ist. Hat daher das Signal $U_1$ z.B. eine Frequenz f = 100 MHz, dann hat das Ausgangssignal des Frequenzvervielfachers 4 eine Frequenz von f = 4 GHz. Als Frequenzvervielfacher 4 wird z.B. ein Baustein mit einem Varactor verwendet (vgl. z.B. M.S. Schumilin, O.W. Golovin in "Radiosendervorrichtungen", Moskau, Hochschule, 1981, S. 155 − 157, Bilder 9.10. oder 9.11).

Nach der Verstärkung des Ausgangssignals des Frequenzvervielfachers 4 im Verstärker 5 tritt das Signal in die Antennenvorrichtung 3 ein, die über ein Koaxkabel od. dgl. mit dem Verstärker 5 verbunden sein kann und vorzugsweise eine Spiral−(Helical−)Antenne enthält (vgl. z.B. D. Nührmann in "Das große Werkbuch der Elektronik", Franzis−Verlag München 1989, S. 2684 − 2688).

Die von der Antennenvorrichtung 3 abgestrahlte elektromagnetische Energie besitzt eine Energiestromdichte von nicht mehr als 100 m Wt pro $cm^2$, vorzugsweise nicht mehr als 10 m Wt pro $cm^2$. Dadurch kann bei einer Bestrahlungsdauer von nicht mehr als zehn Minuten in keinem Fall die höchste zulässige Stufe der Bestrahlung des Menschen im Frequenzbereich bis 30 GHz überschritten werden, da diese bei einer ununterbrochenen Bestrahlungsdauer über eine Stunde hinweg und mehr bei 100 m Wt pro $cm^2$ liegt.

Anstatt zur Behandlung von Bronchialasthma eignet sich die Erfindung vor allem auch zur Behandlung der Dermatose bei Säugetieren. Bei der Behandlung von Bronchialasthma wurden in 89 % aller Fälle selbst dort Erfolge erzielt, wo die Anwendung bekannter Höchstfrequenzvorrichtungen im wesentlichen wirkungslos war. Weitere Erfolge wurden bei der Beeinflussung des Wachstums von Getreidekorn und bei der Stimulation von Pflanzen, Keimen, Embryonen und Säugetieren, z.B. Kühen und Hunden, erzielt.

Die Erfindung ist nicht auf die beschriebenen Ausführungsbeispiele beschränkt, die sich auf vielfache Weise abwandeln lassen. Insbesondere sind die beschriebenen Arten der Frequenz− und Amplitudenmodulation nur als Beispiele aufzufassen. Es wäre beispielsweise auch möglich, die Zahl n ≥ 2 der Kanäle bzw. der Spannungsgeneratoren 14 bis 16 auf eine Zahl zu vergrößern, die wesentlich größer als 2 ist, und in Abhängigkeit davon einen Schalter 9 mit mehr als vier Eingängen sowie eine Schieberegister 28 mit mehr als vier D – Flipflops vorzusehen. Wichtig ist dabei vor allem, daß das von der Antennenvorrichtung 3 abgestrahlte Signal durch Frequenz – und/oder Amplitudenmodulation ständig so verändert wird, daß sich das bestrahlte biologische Objekt bzw. dessen Zellen od. dgl. nicht oder nur langsam der Strahlung anpassen und dadurch deren Wirkung beeinträchtigen oder vollständig verhindern können.

**Patentansprüche**

1. Vorrichtung für die Stimulation des Funktionszustands eines biologischen Objekts, bestehend aus einem UHF – Generator (1), dem eine Modulationseinrichtung (2) zugeordnet und eine Antenneneinrichtung (3) nachgeschaltet ist, dadurch gekennzeichnet, daß die Modulationseinrichtung (2) einen nach einem pseudozufälligen Gesetz arbeitenden Frequenz – und/oder Amplitudenmodulator (6,7) enthält.

2. Vorrichtung nach Anspruch 1, dadurch gekennzeichnet, daß die Modulationseinrichtung (2) unter der Steuerung einer pseudozufällig arbeitenden Steuervorrichtung (8) steht, die ständig wechselnde Modulationen erzeugt.

3. Vorrichtung nach Anspruch 2, dadurch gekennzeichnet, daß die Steuervorrichtung (8) einen Schalter (9) enthält, der einen an die Modulationseinrichtung (2) angeschlossenen Ausgang (10), wenigstens zwei, in zufälligem Wechsel an Spannungsgeneratoren (14,15,16) mit steuerbarer Frequenz anschließbare Eingänge (11,12,13) und wenigstens einen mit einer Schalteinrichtung (18) verbundenen Steuereingang (17) aufweist.

4. Vorrichtung nach Anspruch 3, dadurch gekennzeichnet, daß die Spannungsgeneratoren (14,15,16) mit steuerbarer Frequenz je einen Funktionsgenerator (21,22,23) enthalten, der einen an einen Sägezahn – oder Dreiecksspannungserzeuger (24,25,26) angeschlossenen Steuereingang aufweist.

5. Vorrichtung nach einem der Ansprüche 1 bis 4, dadurch gekennzeichnet, daß die Schalteinrichtung (19) wenigstens zwei Adressenausgänge ($X_1$, $X_2$) aufweist, an denen in zufälliger Reihenfolge binäre "1" – und "0" – Signale erscheinen, und daß der Schalter (9) einen Multiplexer (27) mit wenigstens zwei, mit den Adressenausgängen ($X_1$, $X_2$) verbundenen Steuereingängen (17) enthält.

6. Vorrichtung nach einem der Ansprüche 1 bis 5, dadurch gekennzeichnet, daß der Frequenzmodulator (6) eine die Frequenz des UHF – Generators (1) steuernde und mit dem Ausgang (10) des Schalters (9) verbundene Kapazitätsdiode (Varicap VD) aufweist.

7. Vorrichtung nach einem der Ansprüche 1 bis 6, dadurch gekennzeichnet, daß der UHF – Generator (1) einen Resonanzschwingkreis aufweist, mit dessen Ausgang ein an einen Amplitudenmodulator (7) angeschlossener Schaltkreis verbunden ist.

8. Vorrichtung nach einem der Ansprüche 1 bis 7, dadurch gekennzeichnet, daß die Anordnung so getroffen ist, daß die Dichte der abgestrahlten elektromagnetischen Energie Kleiner als 100 m Wt/cm$^2$ ist.

Fig. 1

EP 0 543 152 A2

Fig. 2

Fig. 3

Fig. 4

Fig. 5

Fig. 6

Fig. 7